(19) Europäisches Patentamt
European Patent Office
Office européen des brevets

(11) Veröffentlichungsnummer : **0 021 221**
**B1**

(12) # EUROPÄISCHE PATENTSCHRIFT

(45) Veröffentlichungstag der Patentschrift : **24.11.82**

(21) Anmeldenummer : **80103205.3**

(22) Anmeldetag : **10.06.80**

(51) Int. Cl.³ : **C 07 C 59/135**, C 07 C 51/58, B 01 J 31/28

(54) **Verfahren zur Dimerisierung von Hexafluorpropenoxid.**

(30) Priorität : **16.06.79 DE 2924385**

(43) Veröffentlichungstag der Anmeldung : **07.01.81 (Patentblatt 81/01)**

(45) Bekanntmachung des Hinweises auf die Patenterteilung : **24.11.82 Patentblatt 82/47**

(84) Benannte Vertragsstaaten : **DE FR GB IT NL**

(56) Entgegenhaltungen : **DE A 2 756 919**

(73) Patentinhaber : **HOECHST AKTIENGESELLSCHAFT**
**Postfach 80 03 20**
**D-6230 Frankfurt/Main 80 (DE)**

(72) Erfinder : **Kühne, Gerhard, Dr.**
**Marienberger Strasse 7**
**D-8263 Burghausen/Salzach (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 021 221 B1

Verfahren zur Dimerisierung von Hexafluorpropenoxid

Die Erfindung betrifft ein Verfahren zur katalytischen Dimerisierung von Hexafluorpropenoxid, gegebenenfalls im Gemisch mit Hexafluorpropen, in Gegenwart eines aprotischen organischen Lösungsmittels bei Temperaturen von − 10 bis + 10 °C und unter autogenem Druck.

Verfahren zur Polymerisation und/oder zur Oligomerisierung von Hexafluorpropenoxid (im folgenden mit HFPO abgekürzt) unter Einsatz von verschiedenen Katalysatoren sind bekannt. Dabei entstehen oligomere und polymere Säurefluoride der Formel

$$CF_3 \cdot CF_2 \cdot CF_2 \cdot O \cdot [CF(CF_3)CF_2 \cdot O]_n \cdot CF(CF_3)C{\genfrac{}{}{0pt}{}{\nearrow O}{\searrow F}} ,$$

worin n Werte im Bereich zwischen 0 und etwa 25 bis 30 annehmen kann. Von diesen Oligomeren ist das Dimere des HFPO (Perfluor-2-n-propoxypropionylfluorid, n = 0) von besonderem Interesse, da es durch Überführung in das Alkalisalz der homologen Carbonsäure und dessen Pyrolyse zu dem technisch interessanten Perfluorpropylperfluorvinylether weiter umgesetzt werden kann. Dieser Ether dient als wichtiges Comonomeres für zahlreiche fluorhaltige Mischpolymerisate. Daher ist die Lenkung der Oligomerisierungsreaktion in Richtung auf einen möglichst hohen Anteil des Dimeren im Oligomerisierungsprodukt besonders erwünscht, um kostspielige Verluste an einer teueren Substanz zu vermeiden.

Bekannt ist (DE-PS 11 48 750) der Einsatz von Aktivkohle oder von energiereicher, ionisierender Strahlung bei der Gasphasenpolymerisation von HFPO. Hierbei werden jedoch nur sehr geringe Anteile an Dimerem neben hohen Anteilen an höheren Oligomeren erhalten, bei Einsatz von Aktivkohle ist außerdem die Reaktion wegen deren schwankendem Gehalt an katalytisch wirksamen Anteil schwer reproduzierbar zu führen. Bekannt ist ferner (DE-PS 15 20 527) die katalytische Wirkung von Alkalimetallfluoriden, Silberfluorid oder Thalliumfluorid, gegebenenfalls in Abmischung mit anderen Alkalimetallhalogeniden, bei der Oligomerisierung von HFPO in polaren organischen Lösungsmitteln. Diese Katalysatoren ergeben zwar eine etwas in Richtung des Dimeren verbesserte, aber immer noch zu breite Molekulargewichtsverteilung der erhaltenen Oligomeren. Alkalimetallfluoride, vor allem das häufig verwendete Cäsiumfluorid, besitzen den zusätzlichen Nachteil, daß sie auch die Oligomerisierung von Hexafluorpropen (im folgenden mit HFP abgekürzt) katalysieren und so zu schwer abtrennbaren Nebenprodukten führen. HFP ist bekanntlich das Ausgangsmaterial für die Herstellung von HFPO und daher in letzterem meist in Anteilen bis zu 30 Gew.-% enthalten. Die Trennung des Gemisches HFP/HFPO ist kostspielig und aufwendig, weshalb es wünschenswert ist, dieses Gemisch direkt für die Oligomerisierung einzusetzen.

Silberfluorid als Katalysator für die genannte Oligomerisierungsreaktion erbringt einen höheren Anteil an Dimerem, es besitzt aber den Nachteil einer sehr geringen Löslichkeit in organischen Lösungsmitteln und ferner einer sehr großen Empfindlichkeit gegen Feuchtigkeit. Silbernitrat, bekannt aus der DE-OS 20 26 669, ist ebenfalls ein relativ billiger und wirksamer Katalysator, jedoch muß bei seinem Einsatz die Bildung und Freisetzung von nitrosen Gasen und, dadurch bedingt, die Bildung von unerwünschten fluorhaltigen Nitrosoverbindungen in Kauf genommen werden. Alle Silberkatalysatoren sind außerdem lichtempfindlich, was zur nachteiligen Ausscheidung von kolloidalem Silber führt.

Bekannte organische funktionelle Verbindungen mit Elementen der V. und VI. Hauptgruppe des Periodischen Systems als Oligomerisierungskatalysatoren für HFPO, wie quartäre Salze des Stickstoffs, Phosphors und Arsens (DE-PS 16 45 114), tertiäre Amine und Aminoxide (DE-PS 16-45 115) und Sulfoniumhalogenide (DE-OS 26 14 332) haben den Nachteil, daß sie organische Nebenprodukte bilden, die nur unter großen Schwierigkeiten aus dem Produktgemisch entfernt werden können und die sich teilweise bei einem kontinuierlichen Verfahren im Reaktionsgefäß anreichern.

Somit besteht nach wie vor ein Bedürfnis nach einem katalytischen Verfahren zur Oligomerisierung von HFPO unter Einsatz eines Katalysators, der möglichst selektiv wirken soll, das heißt insbesondere die Oligomerisierung von HFP nicht katalysiert, der leicht handhabbar ist, der eine hohe Ausbeute an dimerem Produkt neben möglichst wenig Isomerisierungsprodukt und höhere Oligomerisierungsprodukte liefert, der selbst keine unerwünschten und insbesondere schwer abtrennbaren Nebenprodukte ergibt und der schließlich vom Produktgemisch leicht abtrennbar und somit wiederverwendbar sein soll.

Dies kann erreicht werden durch ein Verfahren der eingangs genannten Art, das dadurch gekennzeichnet ist, daß als Katalysator zur Dimerisierung von HFPO, gegebenenfalls im Gemisch mit Hexafluorpropen, in Gegenwart eines aprotischen organischen Lösungsmittels bei Temperaturen von − 10 bis + 10 °C und unter autogenem Druck ein Gemisch, bestehend aus

a) einer Mischung aus einer Kupfer(I)-Verbindung der Formel $Cu_mX$ und einer Kupfer(II)-Verbindung der Formel $Cu_{m/2}X$, wobei X = 0 oder ein Anion und m die Wertigkeit von X bedeutet, im Gewichtsverhältnis 70 : 30 bis 95 : 5 und

b) einem Komplexbildner aus der Gruppe der Nitrile der allgemeinen Formel

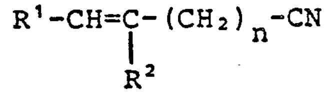

worin $R^1$ und $R^2$, gleich oder verschieden, H, CN oder einen Alkylrest mit 1 bis 6 C-Atomen bedeutet und einer dieser Alkylrest $R^1$ oder $R^2$ gegebenenfalls mit einer CN-Gruppe substituiert sein kann, oder worin $R^1$ und $R^2$ zusammen mit den benachbarten C-Atomen die Bedeutung einer cyclischen Gruppe der Formel

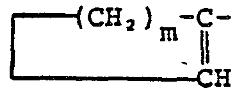

worin m = 3 bis 10 ist, annehmen können und worin ferner n eine ganze Zahl von 0 bis 5 bedeutet, der Isocyanide der allgemeinen Formel $R^3NC$, worin $R^3$ einen Alkylrest, einen Cycloalkylrest, einen Aralkylrest oder einen, gegebenenfalls mit 1 bis 3 $CH_3$-Gruppen substituierten, ein- oder zweikernigen Arylrest bedeutet, der tertiären Phosphine $R^4R^5R^6P$, worin $R^4$, $R^5$ und $R^6$, gleich oder verschieden, einen Alkylrest, einen Cycloalkylrest, einen Aralkylrest oder einen, gegebenenfalls mit 1 bis 3 $CH_3$-Gruppen substituierten, ein- oder zweikernigen Arylrest bedeutet, oder der cyclischen Kohlenwasserstoffe mit 2 oder 3 Doppelbindungen und mit 6 bis 12 C-Atomen im Ring oder in bi- oder tricyclischen Ringsystemen, oder ein Gemisch dieser Komplexbildner eingesetzt wird, wobei das Molverhältnis der Komponente a) zu Komponente b) 1 : 1 bis 1 : 8 beträgt.

Als einzusetzende Salze des einwertigen Kupfers für die Komponente a) sind insbesondere zu nennen die Kupfer(I)-halogenide, wie das Fluorid, Chlorid und Bromid, ferner auch Kupfer(I)-carboxylate, wie beispielsweise das Formiat, Acetat, Propionat, Butyrat, Benzoat, o-Nitrobenzoat, das Monochloracetat, Dichloracetat und das Trifluoracetat des einwertigen Kupfers. Bevorzugt dient als Komponente a) das Kupfer(I)-chlorid und das Kupfer(I)-oxid. Als einzusetzende Salze des zweiwertigen Kupfers für die Komponente a) sind insbesondere zu nennen die Kupfer(II)-halogenide, wie das Fluorid, Chlorid und Bromid, das Kupfer(II)-sulfat, -nitrat, -phosphat, ferner auch Kupfer(II)-carboxylate wie Kupfer(II)-acetat, -propionat, -benzoat und -trifluoracetat. Bevorzugt sind das Kupfer(II)-chlorid, das Kupfer(II)-acetat und das Kupfer(II)-oxid. Das Gewichtsverhältnis von Cu(I)-Verbindung zu Cu(II)-Verbindung in der Komponente a) liegt im Bereich 70 : 30 bis 95 : 5, vorzugsweise 80 : 20 bis 90 : 10.

Die Cu(II)-Salze in der Komponente a) können die gleichen Anionen tragen wie die oben beschriebenen Cu(I)-Salze. Die Gemische können aber auch aus unterschiedlichen Anionen bestehen, wie beispielsweise $CuCl + Cu(CH_3COO)_2$. Auch Gemische von $Cu_2O$ mit Cu(II)-Salz oder CuO mit Cu(I)-Salz sind geeignet.

Die einzusetzenden Komplexbildner der Komponente b) werden aus folgenden Gruppe ausgewählt :
Nitrile der allgemeinen Formel

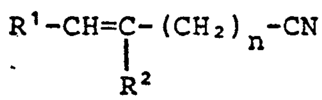

hierin bedeuten $R^1$ und $R^2$, die gleich oder verschieden sein können, Wasserstoff, eine CN-Gruppe oder einen Alkylrest mit 1 bis 6 C-Atomen, wobei einer dieser Alkylreste mit einer CN-Gruppe substituiert sein kann.

$R^1$ und $R^2$ können ferner gemeinsam die Bedeutung einer Alkylengruppe annehmen und somit eine Ringgruppierung

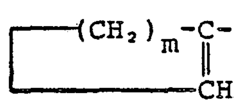

m = 3 bis 10, vorzugsweise 3 bis 6, insbesondere 3, ergibt. Vorzugsweise bedeuten $R^1$ und $R^2$, gleich oder verschieden, Wasserstoff, eine CN-Gruppe oder eine Methyl- oder Ethylgruppe. n kann eine ganze Zahl von 0 bis 5 sein. Vorzugsweise ist n = 0 oder 1. Als Vertreter dieser Gruppe sind beispielsweise zu nennen Allylcyanid, Malein- und Fumarsäuredinitril, 1.1-Dicyanethylen und vorzugsweise Crotonsäurenitril, Methacrylnitril und insbesondere Acrylnitril.

Isocyanide der Formel $R^3NC$, hierin bedeutet $R^3$ einen geradkettigen oder verzweigten Alkylrest, vorzugsweise einen solchen mit 3 bis 5 C-Atomen, einen Cycloalkylrest, vorzugsweise einen mit 5 bis 8 C-Atomen im Ring, insbesondere einen Cyclohexylrest. Ferner bedeutet $R^3$ einen Aralkylrest, vorzugsweise einen Benzylrest oder einen ein- oder zweikernigen Arylrest, der gegebenenfalls mit 1 bis 3 $CH_3$-Gruppen substituiert sein kann. Letzterer Rest ist vorzugsweise ein Phenyl-, ein Toluyl-, ein Xylyl-, ein Mesityl- oder ein Naphthylrest. Vertreter dieser Gruppe sind beispielsweise 2-, 3- und 4-Methylphenylisocyanid, 2.3-, 2.4- und 2.5-Dimethylphenylisocyanid, 1- und 2-Naphthylisocyanid sowie vorzugsweise Phenylisocyanid,

n-Propylisocyanid, tert.-Butylisocyanid und insbesondere Cyclohexylisocyanid.

Tertiäre Phosphine der Formel $R^4R^5R^6P$, worin $R^3$, $R^4$ und $R^5$, die verschieden oder vorzugsweise gleich sein können, einen geradkettigen oder verzweigten Alkylrest, vorzugsweise einen solchen mit 1 bis 5 C-Atomen und insbesondere mit 3 bis 5 C-Atomen, einen Cycloalkylrest, vorzugsweise einen Cyclohexylrest, einen Aralkylrest, vorzugsweise einen Benzylrest, sowie einen ein- oder zweikernigen Arylrest, wobei der letztere gegebenenfalls mit 1 bis 3 $CH_3$-Gruppen substituiert sein kann, bedeuten. Dieser Arylrest ist vorzugsweise ein Phenyl- oder ein Toluylrest. Beispielsweise sind aus dieser Gruppe zu nennen Trimethyl-, Triethyl-, Tripropyl-, Triisopropyl-, Tributyl-, Triisobutyl-, Tri-tert.-butylphosphin, ferner Tricyclohexyl-, Tribenzyl-, Tri(o-, p- oder m-)toluylphosphin und insbesondere Triphenylphosphin.

Schließlich kommen als Komplexbildner noch cyclische Kohlenwasserstoffe mit 2 oder 3 Doppelbindungen und mit 6 bis 12 C-Atomen im Ring in Frage, wobei dieses Ringgerüst ein Tricyclus, ein Bicyclus oder vorzugsweise ein Monocyclus ist. Ausgenommen ist Benzol. Besonders bevorzugte Vertreter sind das Cyclooctadien-1.5 und das Cyclododecatrien-1.5.9.

Die genannten Kompexbildner können auch in Form von Gemischen eingesetzt werden, wobei die einzelnen Vertreter in einem solchen Gemisch sowohl aus verschiedenen der obengenannten Gruppen, vorzugsweise aber aus der gleichen Gruppe stammen können.

Das Gemisch aus Cu(I)-Verbindung und Cu(II)-Verbindung der Komponente a) und der Komplexbildner der Komponente b) werden im erfindungsgemäßen Katalysatorsystem in einem Molverhältnis von 1 : 1 bis 1 : 8, vorzugsweise 1 : 1 bis 1 : 4, eingesetzt.

Die Menge der Komponente a) soll je nach der Größe des Ansatzes und der Kühlkapazität des Reaktors zwischen 0,1 und 3 Mol-%, bezogen auf die eingesetzte Menge an HFPO, liegen. Bei den in der Praxis verwendeten Ansätzen von 25 bis 50 kg Reaktionsmischung liegt die eingesetzte Menge an Komponente a) zwischen 0,8 und 1,0 Mol-%.

Das erfindungsgemäße Verfahren wird in Gegenwart eines aprotischen organischen Lösungsmittels durchgeführt, das ein ausreichendes Lösevermögen für das Katalysatorsystem besitzen soll und mit HFPO und den Reaktionsprodukten nicht reagiert. Dies sind im wesentlichen polare organische Lösungsmittel (apolare können im Gemisch mit polaren Lösungsmitteln eingesetzt werden), wie beispielsweise Acetonitril, Propionitril, Benzonitril, Nitrobenzol, Pyridin, Chinolin, N-Methylpyrrolidon, Tetramethylharnstoff, Dioxan, Nitroethan, Dimethylformamid, Dimethylacetamid sowie Dialkylsulfone und Sulfolane. Ferner sind zu nennen die Dialkylether (vorzugsweise mit Methyl-, Ethyl- und Propylresten) von Ethylen- und Propylenglykol sowie von den entsprechenden Di-, Tri- und Polyglykolen einschließlich der Mischpolyglykole mit Ethylenoxid- und Propylenoxideinheiten, ebenso auch niedrigsiedende, insbesondere aliphatische Ester.

Die genannten Lösungsmittel oder auch Gemische solcher Lösungsmittel werden in einer Menge von 5 bis 30 Gew.-%, vorzugsweise 5 bis 15 Gew.-%, bezogen auf die gesamte Reaktionsmischung, eingesetzt.

Das erfindungsgemäße Verfahren wird durchgeführt bei Temperaturen zwischen − 10 und + 10 °C, vorzugsweise zwischen − 5 und + 5 °C. Die genannten Temperaturbereiche gelten im Mittel und sind nicht absolut kritisch, sondern diese Temperaturen können auch während der Reaktion geringfügig überschritten oder unterschritten werden. Man kann die Temperatur im genannten Bereich auch während der Reaktion ansteigen oder abfallen lassen. Bei den genannten Reaktionstemperaturen zwischen − 10 und + 10 °C baut sich im Reaktionsgefäß durch Einschleusen von HFPO und durch die Gegenwart der Katalysatorlösung ein Druck von 2 bis 4,5 bar auf, vorzugsweise wird bei 2,5 bis 3,5 bar an autogenem Druck gearbeitet. Da HFP bei Normaldruck einen Siedepunkt von − 28,3 °C hat (HFPO − 28,5 °C), ändern sich diese Verhältnisse beim Vorliegen von Gemischen von HFPO/HFP praktisch nicht.

Die technische Durchführung des erfindungsgemäßen Dimerisierungsverfahrens erfolgt zweckmäßigerweise in einem Druckrührbehälter aus Edelstahl (beispielsweise mit einem Fassungsvermögen von 50 bis 100 l), der mit Rührer, Bodenventil und Kühlmantel sowie den entsprechenden Zuleitungen versehen ist. Die Temperatur sollte durch Temperaturmeßstellen sowohl im Behälterinnenraum, im Mantel und im Gasraum kontrolliert werden. Es können Blatt-, Anker- oder Impellerrührer verwendet werden, der Einbau eines Stromstörers ist zwecks optimaler Durchmischung empfehlenswert. Die Reaktion kann ebenso auch in einem Schüttelautoklaven durchgeführt werden. Die Zuleitung für das HFPO bzw. das HFPO/HFP-Gemisch ist mit einer Druckschleuse versehen, durch die kontinuierlich oder portionsweise zudosiert werden kann.

Die Katalysatorlösung, das heißt das Gemisch aus Kupfer(I)- und Kupfer(II)-Verbindung der Komponente a), dem Komplexbildner und dem organischen Lösungsmittel, wird zweckmäßigerweise vorgemischt und dann vorgelegt oder portionsweise bzw. kontinuierlich dem Reaktionsgefäß über eine entsprechende Zuleitung zugeführt. Es können jedoch auch die Mischung aus Kupfer(I)- und Kupfer(II)-Verbindung und Komplexbildner einerseits und das Lösungsmittel andererseits oder die Mischung aus Kupfer(I)- und Kupfer(II)-Verbindung und Lösungsmittel einerseits und der Komplexbildner andererseits getrennt zugegeben werden.

Der Reaktor wird vor Beginn der Reaktion unter Rühren mit Hilfe des Kühlmantels auf eine Temperatur um 0 °C abgekühlt. Sodann wird das HFPO oder das HFPO/HFP-Gemisch portionsweise oder kontinuierlich so zudosiert, daß die Temperatur in der flüssigen Phase des Reaktors zwischen etwa − 10 und + 10 °C, vorzugsweise zwischen − 5 und + 5 °C, eingestellt wird.

Nach Beendigung der Zudosierung wird bei Temperaturen um 0 °C noch 1 bis 2 Stunden

nachgerührt, sodann wird der Kessel entspannt. Das Abgasgemisch wird über Kältefallen mit Temperaturen von ca. – 60 °C kondensiert.

Nach Abstellen des Rührers trennt sich das flüssige Gemisch in zwei Phasen, wobei die obere Phase nahezu ausschließlich die Katalysatorbestandteile und das organische Lösungsmittel enthält, während das dimere Reaktionsprodukt und die entstandenen oligomeren Nebenprodukte die Unterphase bilden. Die obere Phase kann im Reaktionsgefäß verbleiben und nach Ergänzung der notwendigen Menge an Katalysator sofort für die nächste Reaktion eingesetzt werden, die Unterphase wird über ein Bodenventil abgelassen.

Der technische Vorteil des im erfindungsgemäßen Verfahren eingesetzten Katalysatorsystems liegt insbesondere in seiner einfachen Wiederverwendung für die gleiche Umsetzung, das heißt, er ist nicht nur für die Chargenfahrweise, sondern auch für eine Art Kaskadenfahrweise mit Absitzbehälter geeignet, aus welchem die Katalysatorlösung in den Reaktionskessel zurückgeführt wird. Wegen der langen Lebensdauer dieses Katalysatorsystems und des geringen Verbrauchs während der Reaktion brauchen dem Reaktionskessel vor jedem neuen Ansatz nur untergeordnete Mengen frische Katalysatorlösung zudosiert zu werden.

Auch wurde festgestellt, daß das erfindungsgemäße Katalysatorsystem bei einem wiederholten Einsatz zu einer Verschiebung der ohnehin günstigen Molekulargewichtsverteilung in Richtung des Dimeren führt, so daß das erfindungemäße Verfahren ganz besonders für eine kontinuierliche Fahrweise geeignet ist. Es bildet sich dann ein Gleichgewichtszustand heraus, in dem das gewünschte Dimere in hohem Anteil gebildet wird.

Durch die Wiedereinsetzbarkeit unterscheidet sich das erfindungsgemäße Katalysatorsystem vorteilhaft insbesondere von dem hinsichtlich der erzielten Oligomerenverteilung auch günstigen Silber-Katalysatoren, da diese entweder als Agf eingesetzt werden oder im Verlauf der Reaktion AgF bilden. Dieses ist jedoch in allen gebräuchlichen organischen Lösungsmitteln so schwer löslich, daß es darin lediglich in feinster Form suspendiert bzw. dispergiert werden kann. Dieser überwiegende, ungelöste, lediglich suspendierte Anteil des Silber-Katalysators verteilt sich bei der Phasentrennung im Anschluß an die Reaktion gleichmäßig über die Lösungsmittelphase und die Produktphase und wird demgemäß mit dem Produkt abgeführt. Er muß daraus wiedergewonnen werden, was zeitraubend und umständlich ist.

Es wurde gefunden, daß ein Zusatz von Cu(II)-Salzen innerhalb der Komponente a) eine Steigerung des Dimerenanteils im Oligomerengemisch erbringt, was überrascht, da Cu(II)-Salze und Cu(II)-Oxid für sich allein in Gegenwart der Komplexbildner der Komponente b) überhaupt keine katalytische Wirksamkeit bei der Oligomerisierung von HFPO aufweisen.

Soweit Gemische von HFPO und HFP eingesetzt werden, ist es ferner von großem Vorteil, daß eine Dimerisierung oder Oligomerisierung von HFP beim erfindungsgemäßen Verfahren praktisch nicht beobachtet wird. Daher kann der gesamte Gehalt an HFP mit den flüchtigen Verbindungen problemlos vom flüssigen Reaktionsprodukt, das hauptsächlich aus dem HFPO-Dimeren besteht, abgetrennt werden, und es bedarf keinerlei zusätzlicher aufwendiger Trennungsmethoden.

Die nachfolgenden Beispiele sollen die Erfindung erläutern.

## Beispiel 1

In einem trockenen Druckrührautoklaven von 1 l Inhalt wird eine Katalysatorlösung, bestehend aus

8,0 g CuCl (= 2,0 Mol-%, bezogen auf HFPO)
2,0 g $CuCl_2$ (= 0,5 Mol-%, bezogen auf HFPO)
20,0 g Acrylnitril
100,0 ml Acetonitril

vorgelegt.

Der Autoklav wird auf 0 °C heruntergekühlt und dann mit 740 g eines Gemisches von HFPO und HFP (HFPO-Gehalt 90 Gew.-%) portionsweise in Dosiermengen von je ca. 50 ml über eine Schleuse beschickt. Die Dosierung wird so gesteuert, daß die Autoklaveninnentemperatur sich in einem Intervall von 0 bis + 2 °C bewegt. Die Gesamtdosierzeit beträgt 3 Stunden. Nach einstündiger Nachreaktionszeit bei 0 °C unter Rühren wird das Autoklavenventil geöffnet und die flüchtigen Bestandteile in einer Kühlfalle kondensiert. 90 g aufgefangenes Kondensat bestehen aus :

75 g HFP (80 Gew.-%)
12 g $CF_3CF_2COF$ (16 Gew.-%)
3 g sonstige nicht identifizierte Verbindungen (4 Gew.-%).

Der flüssige Inhalt wird mit Stickstoff aus dem Autoklaven gedrückt und die Oberphase, die praktisch nur aus der Katalysatorlösung besteht, von der Unterphase abgetrennt. Die Unterphase macht 650 g aus und besteht aus :

550,0 g = 84,6 Gew.-% Dimerem,

25,3 g = 3,9 Gew.-% Trimerem,
74,7 g = 11,5 Gew.-% höheren Oligomeren und sonstigen, nicht näher identifizierten Verbindungen.

Auf das eingesetzte HFPO bezogen werden somit 82,7 % Dimeres gewonnen.

Ein in gleicher Weise durchgeführter Ansatz, aber mit dem Verhältnis Cu(I)-Salz : Cu(II)-Salz = 90 : 10, das heißt

9,0 g CuCl (= 2,25 Mol-%, bezogen auf HFPO)
1,0 g $CuCl_2$ (= 0,25 Mol-%, bezogen auf HFPO)

liefert einen Anteil von 81,0 Gew.-% Dimerem, bezogen auf eingesetztes HFPO.

Beispiel 2

Der Versuch des Beispiels 1 wird mit folgender Katalysatormischung, aber unter sonst gleichen Bedingungen, wiederholt :

4 g CuCl (= 1,0 Mol-%, bezogen auf HFPO)
1 g $CuCl_2$ (= 0,25 Mol-%, bezogen auf HFPO)
5 g Cyclohexylisonitril
50 ml Acetonitril.

Nach einer Gesamtreaktionszeit von 4 Stunden bei 0 bis + 2 °C wird das Autoklavenventil geöffnet und die flüchtigen Bestandteile in einer Kältefalle kondensiert. 155 g aufgefangenes Kondensat bestehen aus :

75 g HFP (48,0 Gew.-%)
50 g $CF_3CF_2COF$ (32,3 Gew.-%)
30 g sonstige, nicht identifizierte Verbindungen (19,7 Gew.-%).

Der flüssige Inhalt wird aus dem Autoklaven gedrückt und zur Phasentrennung stehen gelassen. Die Unterphase, die 595 g ausmacht, besteht aus :

475 g = 80 Gew.-% Dimerem,
78 g = 13 Gew.-% Trimerem,
42 g = 7 Gew.-% höheren Oligomeren und sonstigen, nicht identifizierten Verbindungen.

Bezogen auf eingesetztes HFPO werden 71,2 % Dimeres gebildet.

Beispiel 3

Der Versuch des Beispiels 1 wird mit folgender Katalysatormischung, aber unter sonst gleichen Bedingungen, wiederholt :

8,0 g CuCl (= 2,0 Mol-%, bezogen auf HFPO)
2,0 g $CuCl_2$ (= 0,5 Mol-%, bezogen auf HFPO)
10 ml Cyclododecatrien-1.5.9
100 ml Acetonitril.

Nach einer Gesamtreaktionszeit von 4 Stunden bei einer Temperatur von 0 bis + 2 °C wird das Autoklavenventil geöffnet und die flüchtigen Bestandteile abgelassen, die hier nicht näher analysiert wurden. Der flüssige Inhalt wird gesammelt und die Phasen voneinander getrennt. Die Oberphase stellt praktisch reine Katalysatorlösung dar. Die Unterphase von insgesamt 650 g besteht aus :

490 g = 75,4 Gew.-% Dimerem,
90 g = 13,8 Gew.-% Trimerem,
70 g = 10,8 Gew.-% höheren Oligomeren und anderen, nicht identifizierten Substanzen.

Bezogen auf eingesetztes HFPO beträgt die Ausbeute an Dimerem 73,6 %.

Beispiel 4

In einem sorgfältig getrockneten Druck-Rührautoklaven aus Edelstahl von 300 ml Inhalt mit Magnetrührer, Kühlvorrichtung und Sicherheitsventil wird eine Lösung von 1,8 g Cu(I)-acetat (1,88 Mol-%, bezogen auf HFPO), 0,2 g Cu(II)-acetat (0,14 Mol-%, bezogen auf HFPO) und 10,3 g Cyclododecatrien-1.5.9, in 30 ml Acetonitril innig vermischt, vorgelegt. Anschließend werden aus einer Schleuse 140 g HFPO/HFP-Gemisch mit einem Gehalt an HFPO von 93 Gew.-% zudosiert.

Unter Rühren wird die Reaktion bei 0 °C 18 Stunden durchgeführt. Danach wird das Ventil am Autoklaven geöffnet und ausgegast. Die in einer Kältefalle aufgefangene Menge beträgt 12,0 g und ist in der Zusammensetzung zu 80 % Perfluorpropionsäurefluorid.

Nach dem Abtrennen der Oberphase werden 128 g Reaktionsprodukt erhalten, die sich wie folgt zusammensetzen :

92,0 g = 71,8 Gew.-% Dimeres,
24,5 g = 19,1 Gew.-% Trimeres,
11,5 g = 9,1 Gew.-% höhere Oligomere und andere, nicht identifizierte Produkte.

Die Ausbeute an Dimerem beträgt demnach 70,6 %, bezogen auf eingesetztes HFPO.

## Beispiel 5

In dem in Beispiel 4 beschriebenen Druckrührautoklaven werden 1,8 g $Cu_2O$ (1,61 Mol-%, bezogen auf HFPO), 0,2 g Cu(II)-acetat (0,14 Mol-%, bezogen auf HFPO), 7,0 ml Acrylnitril und 25 ml Acetonitril vorgelegt und mit dem Magnetrührer 30 Minuten innig durchmischt. Anschließend werden aus einer Schleuse 140 g HFPO/HFP-Gemisch mit einem Gehalt an HFPO von 93 Gew.-% portionsweise innerhalb von 30 Minuten bei 0 bis + 2 °C zudosiert, und diese Temperatur wird unter Rühren und in einem Thermostatbad 16 Stunden gehalten. Danach wird das Ventil am Autoklaven geöffnet und ausgegast. Die in einer Kältefalle aufgefangene Gasmenge beträgt 10 g und ist in der Zusammensetzung neben HFP zu 30 % Perfluorpropionsäurefluorid. Nach dem Abtrennen der Oberphase werden 130 g Reaktionsprodukt erhalten, das sich wie folgt zusammensetzt :

100,0 g = 77,0 Gew.-% Dimeres,
5,5 g = 4,2 Gew.-% Trimeres,
24,5 g = 18,8 Gew.-% höhere Oligomere und andere, nicht identifizierte Substanzen.

Die Ausbeute an Dimerem, bezogen auf eingesetztes HFPO, beträgt demnach 77,0 %.

## Beispiel 6

In dem in Beispiel 4 beschriebenen Druckrührautoklaven wird eine Lösung, bestehend aus 1,5 g CuCl (1,94 Mol-%, bezogen auf HFPO), 0,17 g $CuCl_2 \cdot 2H_2O$ (0,13 Mol-%, bezogen auf HFPO), 8,6 ml Crotonnitril und 25 ml Acetonitril vorgelegt. Anschließend werden aus einer Schleuse 140 g HFPO/HFP-Gemisch mit einem Gehalt von 93 Gew.-% HFPO portionsweise innerhalb von 30 Minuten bei 0 bis + 2 °C zudosiert, und diese Temperatur wird unter Rühren und in einem Thermostatbad 16 Stunden gehalten. Danach wird das Ventil am Autoklaven geöffnet und ausgegast. Die in einer Kältefalle aufgefangene Gasmenge beträgt 25,0 g und besteht neben HFP zu 60 % aus Perfluorpropionylfluorid.

Nach Abtrennung der Oberphase werden 115 g Reaktionsprodukt gewonnen, das sich wie folgt zusammensetzt :

91,5 g = 79,5 Gew.-% Dimeres,
13,4 g = 11,7 Gew.-% Trimeres,
10,1 g = 8,8 Gew.-% höhere Oligomere und andere, nicht identifizierte Substanzen.

Die Ausbeute an Dimerem, bezogen auf eingesetztes HFPO, beträgt demnach 70,2 %.

## Beispiel 7

In dem in Beispiel 4 beschriebenen Druckrührautoklaven wird eine Katalysatorlösung von 1,5 g CuCl (1,94 Mol-%, bezogen auf HFPO), 0,17 g $CuCl_2 \cdot 2H_2O$ (0,13 Mol-%, bezogen auf HFPO), 7,5 ml Methacrylnitril und 25 ml Acetonitril vorgelegt. Anschließend werden aus einer Schleuse 140 g HFPO/HFP-Gemisch mit einem Gehalt von 93 Gew.-% HFPO portionsweise innerhalb von 30 Minuten bei 0 bis + 2 °C zudosiert, und diese Temperatur wird unter Rühren und in einem Thermostatbad 16 Stunden gehalten. Danach wird das Ventil im Autoklaven geöffnet und ausgegast. Die in einer Kältefalle aufgefangene Gasmenge beträgt 22,0 g und besteht neben HFP zu 50 % aus Perfluorpropionylfluorid.

Nach Abtrennung der Oberphase werden 118 g Reaktionsprodukt gewonnen, das sich wie folgt zusammensetzt :

98,0 g = 83,0 Gew.-% Dimeres,
8,8 g = 7,5 Gew.-% Trimeres,
11,2 g = 9,5 Gew.-% höhere Oligomere und andere, nicht identifizierte Substanzen.

Die Ausbeute an Dimerem, bezogen auf eingesetztes HFPO, beträgt demnach 75,4 %.

Beispiel 8

In dem in Beispiel 4 beschriebenen Druckrührautoklaven wird eine vorher zubereitete Lösung von 1,8 g Cu(I)-trifluoracetat (1,30 Mol-%, bezogen auf HFPO), 0,17 g $CuCl_2 \cdot 2H_2O$ (0,13 Mol-%, bezogen auf HFPO), 6,5 g Cyclooctadien-1.5 und 25 ml Acetonitril vorgelegt. Anschließend werden aus einer Schleuse 140 g HFPO/HFP-Gemisch mit einem Gehalt von 93 Gew.-% HFPO portionsweise innerhalb von 30 Minuten bei 0 bis + 2 °C zudosiert, und diese Temperatur wird unter Rühren und in einem Thermostatbad 16 Stunden gehalten. Danach wird das Ventil am Autoklaven geöffnet und ausgegast. Die in einer Kältefalle aufgefangene Gasmenge beträgt 14,0 g und besteht neben HFP zu 30 % aus Perfluorpropionylfluorid.

Nach Abtrennung der Oberphase werden 126 g Reaktionsprodukt gewonnen, das sich wie folgt zusammensetzt :

94,0 g = 74,6 Gew.-% Dimeres,
17,5 g = 13,9 Gew.-% Trimeres,
14,5 g = 11,5 Gew.-% höhere Oligomere und andere, nicht identifizierte Substanzen.

Die Ausbeute an Dimerem, bezogen auf eingesetztes HFPO, beträgt demnach 72,3 %.

Beispiel 9

In der Apparatur gemäß Beispiel 4 wird eine Lösung aus 1,8 g Cu(I)-acetat (1,88 Mol-%, bezogen auf HFPO), 0,2 g Cu(II)-acetat (0,14 Mol-%, bezogen auf HFPO), 6,2 g Triphenylphosphin und 30 ml Acetonitril vorgelegt. Anschließend werden aus einer Schleuse 140 g HFPO/HFP-Gemisch mit einem Gehalt von 93 Gew.-% HFPO portionsweise innerhalb von 30 Minuten bei 0 bis + 2 °C zudosiert, und diese Temperatur wird unter Rühren 16 Stunden gehalten. Darauf wird das Ventil am Autoklaven geöffnet und ausgegast. Die in einer Kältefalle aufgefangene Gasmenge beträgt 13,0 g und besteht neben HFP zu 40 % aus Perfluorpropionylfluorid.

Der flüssige Anteil des Reaktionsproduktes läßt sich in Ober- und Unterphase trennen. Die Unterphase mit dem gesuchten Umsetzungsprodukt macht 127 g aus und setzt sich wie folgt zusammen :

103,0 g = 81,1 Gew.-% Dimeres,
11,3 g = 8,9 Gew.-% Trimeres,
12,7 g = 10,0 Gew.-% höhere Oligomere und andere, nicht identifizierte Substanzen.

Die Ausbeute an Dimerem, bezogen auf eingesetztes HFPO, beträgt demnach 79,2 %.

Mehrfacher Einsatz des Katalysatorsystems

Alle in den Beispielen 1 bis 9 gesammelten Oberphasen wurden getrennt für sich noch einmal im 300 ml-Autoklaven in einer Menge von 25 ml vorgelegt und bei 0 bis + 2 °C innerhalb von 30 Minuten 140 g HFPO/HFP zudosiert. Der Reaktorinhalt wurde unter Rühren 16 Stunden bei 0 bis + 2 °C im Thermostatbad belassen, anschließend ausgegast und in einer Kältefalle gesammelt. Der gasförmige Anteil bewegte sich bei allen Versuchen zwischen 10 und 30 g. Der Anteil an Dimerem hatte sich bei allen Ansätzen um 2 bis 5 % gesteigert.

Die Oberphase des Reaktionsgemisches kann noch 5- bis 6mal mindestens zur wiederholten Oligomerisierung von HFPO eingesetzt werden und liefert dabei eine ähnlich hohe Dimerenausbeute wie im ersten Wiederholungsansatz des Katalysatorsystems.

Ab dem vierten Wiederholungsansatz ist es zweckmäßig, 1 bis 5 % des eingesetzten Cu-Salzes zu ergänzen.

Beispiel 10

In der Versuchsapparatur gemäß Beispiel 4 wird eine vorher zubereitete Lösung von 1,5 g CuCl (1,94 Mol-%, bezogen auf HFPO), 0,17 g $CuCl_2 \cdot 2H_2O$ (0,13 Mol-%, bezogen auf HFPO), 9,0 ml tert.-Butylisocyanid und 25 ml Acetonitril vorgelegt.

Anschließend werden aus einer Schleuse 140 g HFPO/HFP-Gemisch mit einem Gehalt von 93 Gew.-% HFPO portionsweise innerhalb von 30 Minuten bei 0 bis 2 °C zudosiert, und diese Temperatur wird unter Rühren in einem Thermostatbad 16 Stunden gehalten. Daran anschließend wird das Ventil am Autoklaven geöffnet und ausgegast. Die in einer Kältefalle aufgefangene Gasmenge beträgt 15,0 g und besteht neben HFP zu 35 % aus Perfluorpropionylfluorid.

Nach Abtrennung der Oberphase werden 125 g Reaktionsprodukt gewonnen, das sich wie folgt zusammensetzt :

95,5 g = 76,4 Gew.-% Dimeres,
18,0 g = 14,4 Gew.-% Trimeres,
11,5 g = 9,2 Gew.-% höhere Oligomere und andere, nicht identifizierte Substanzen.

Die Ausbeute an Dimerem, bezogen auf eingesetztes HFPO, beträgt demnach 73,5 %.

## Beispiele 11 bis 20

In der in Beispiel 4 beschriebenen Apparatur werden die in der nachfolgenden Tabelle vermerkten Versuche durchgeführt. Die in Spalte 2 verzeichneten Komplexbildner werden in Kombination mit den jeweils zugehörigen Mischungen aus Cu(I)- und Cu(II)-Verbindung des in Spalte 3 angegebenen Beispiels für die Oligomerisierungsreaktion eingesetzt. Die Molverhältnisse der Komponenten und Versuchsbedingungen sind diejenigen des in Spalte 3 vermerkten, vorangegangenen Beispiels:

Tabelle

| Beispiel | Eingesetzter Komplexbildner | Sonstige Versuchsbedingung wie in Beispiel | Ausgegaster Anteil g | Gesamtmenge in g an Oligomeren in der Unterphase | Zusammensetzung in Gew.-% Dimeres | Trimeres | höhere Oligomere |
|---|---|---|---|---|---|---|---|
| 11 | Cyclohexennitril-3 | 4 | 20 | 120 | 75,0 | 8,4 | 16,6 |
| 12 | Fumarsäuredinitril | 6 | 16 | 124 | 76,6 | 12,1 | 11,3 |
| 13 | Allylcyanid | 4, mit der Ausnahme, daß anstelle von Cu(I)-acetat 1,8 g Cu(I)-benzoat eingesetzt werden | 24 | 116 | 79,4 | 11,2 | 9,4 |
| 14 | Benzylisocyanid | 6 | 17 | 123 | 73,2 | 16,3 | 10,5 |
| 15 | Toluylisocyanid | 9 | 22 | 118 | 79,8 | 13,6 | 6,6 |
| 16 | Phenylisonitril | 8 | 18 | 122 | 77,0 | 14,8 | 8,2 |
| 17 | Tri-n-butylphosphin | 9 | 25 | 115 | 77,5 | 9,5 | 13,0 |
| 18 | Tri-cyclohexylphosphin | 4 | 24 | 116 | 79,3 | 6,9 | 13,8 |
| 19 | Tri-benzylphosphin | 9 | 18 | 122 | 77,1 | 14,7 | 8,2 |
| 20 | Dimethylphenylphosphin | 9 | 26 | 114 | 78,2 | 9,6 | 12,2 |

**Anspruch**

Verfahren zur katalytischen Dimerisierung von Hexafluorpropenoxid, gegebenenfalls im Gemisch mit Hexafluorpropen, in Gegenwart eines aprotischen organischen Lösungsmittels bei Temperaturen von − 10 bis + 10 °C und unter autogenem Druck, dadurch gekennzeichnet, daß als Katalysator ein Gemisch, bestehend aus

a) einer Mischung aus einer Kupfer(I)-Verbindung der Formel $Cu_mX$ und einer Kupfer(II)-Verbindung der Formel $Cu_{m/2}X$, wobei $X = 0$ oder ein Anion ist und m die Wertigkeit von X bedeutet, im Gewichtsverhältnis 70 : 30 bis 95 : 5, und

b) einem Komplexbildner aus der Gruppe der Nitrile der allgemeinen Formel

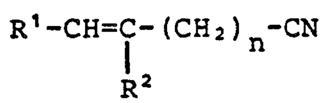

worin $R^1$ und $R^2$, gleich oder verschieden, H, CN oder einen Alkylrest mit 1 bis 6 C-Atomen bedeutet und einer dieser Alkylreste $R^1$ oder $R^2$ gegebenenfalls mit einer CN-Gruppe substituiert sein kann, oder worin $R^1$ und $R^2$ zusammen mit den benachbarten C-Atomen die Bedeutung einer cyclischen Gruppe der Formel

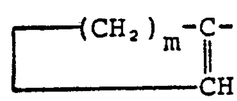

worin m = 3 bis 10 ist, annehmen, und worin ferner n eine ganze Zahl von 0 bis 5 bedeutet, der Isonitrile der allgemeinen Formel

$$R^3NC,$$

worin $R^3$ einen Alkylrest, einen Cycloalkylrest, einen Aralkylrest oder einen, gegebenenfalls mit 1 bis 3 $CH_3$-Gruppen substituierten, ein- oder zweikernigen Arylrest bedeutet, der tertiären Phosphine $R^4R^5R^6P$, worin $R^4$, $R^5$ und $R^6$, gleich oder verschieden, einen Alkylrest, einen Cycloalkylrest, einen Aralkylrest oder einen, gegebenenfalls mit 1 bis 3 $CH_3$-Gruppen substituierten, ein- oder zweikernigen Arylrest bedeutet, oder der cyclischen Kohlenwasserstoffe mit 2 oder 3 Doppelbindungen und mit 6 bis 12 C-Atomen im Ring oder in bi- oder tricyclischen Ringsystemen mit Ausnahme von Benzol, oder Gemischen dieser Komplexbildner eingesetzt wird, wobei das Molverhältnis der Komponente a) zu Komponente b) 1 : 1 bis 1 : 8 beträgt.

## Claim

A process for the catalytic dimerization of hexafluoropropene oxide, optionally in admixture with hexafluoropropene, in the presence of an aprotic organic solvent at temperatures of − 10 to + 10 °C and under autogenous pressure, characterised in that the catalyst employed is a composition containing

a) a mixture of a copper(I) compound of the formula $Cu_mX$ and a copper(II) compound of the formula $Cu_{m/2}X$, wherein X = 0 or an anion and m denotes the valency of X, in the weight ratio of 70 : 30 to 95 : 5 and

b) a complex-forming agent from the group of the nitriles of the general formula

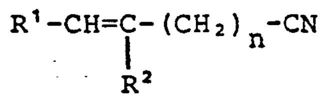

wherein $R^1$ and $R^2$, which are identical or different, denote H, CN or an alkyl radical of 1 to 6 C atoms and one of these alkyl radicals $R^1$ or $R^2$ can optionally be substituted by a CN group, and wherein $R^1$ and $R^2$ can also be linked together with the adjacent C atoms to form a cyclic group of the formula

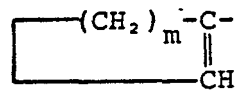

wherein m is an integer of from 3 to 10 and wherein furthermore n denotes an integer from 0 to 5, the isocyanides of the general formula

$$R^3NC,$$

wherein $R^3$ denotes an alkyl radical, a cycloalkyl radical, an aralkyl radical or a mononuclear or binuclear aryl radical which is optionally substituted by 1 to 3 $CH_3$ groups, the tertiary phosphines of the general formula $R^4R^5R^6P$, wherein $R^4$, $R^5$ and $R^6$, which may be identical or different, denote an alkyl radical, a cycloalkyl radical, an aralkyl radical or a mononuclear or binuclear aryl radical which is optionally substituted by 1 to 3 $CH_3$ groups, or the cyclic hydrocarbons with 2 or 3 double bonds and with 6 to 12 C atoms in the ring, or in bicyclic or tricyclic ring systems, or mixtures of these complex-forming agents, the molar ratio of component a) to component b) being 1 : 1 to 1 : 8.

## Revendication

Procédé pour dimériser catalytiquement l'oxyde d'hexafluoropropène, éventuellement en mélange avec de l'hexafluoropropène, en présence d'un solvant organique aprotique, à des températures de − 10 à + 10 °C et sous la pression autogène, procédé caractérisé en ce qu'on utilise comme catalyseur un mélange constitué :

a) d'un mélange d'un composé de cuivre(I) de formule $Cu_mX$ et d'un composé de cuivre(II) de formule $Cu_{m/2}X$, formules dans lesquelles X représente 0 ou un anion et m est égal à la valence de X, dans un rapport pondéral compris entre 70 : 30 et 95 : 5, et

b) d'un formateur de complexes pris dans l'ensemble constitué par les nitriles répondant à la formule générale

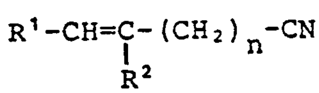

dans laquelle $R^1$ et $R^2$ représentent chacun, indépendamment l'un de l'autre, H, CN ou un radical alkyle contenant de 1 à 6 atomes de carbone et l'un de ces radicaux alkyles $R^1$ et $R^2$ peut porter un groupe CN comme substituant, ou dans laquelle $R^1$ et $R^2$ peuvent représenter ensemble et avec les atomes de carbone voisins un radical cyclique de formule :

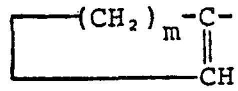

dans lequel m est un nombre de 3 à 10, et n représente un nombre entier de 0 à 5, les isocyanures (« isonitriles ») répondant à la formule générale

$R^3NC$

dans laquelle $R^3$ représente un radical alkyle, cycloalkyle ou aralkyle ou un radical aryle à 1 ou 2 noyaux, éventuellement porteur de 1 à 3 radicaux $-CH_3$, les phosphines tertiaires répondant à la formule $R^4R^5R^6P$ dans laquelle $R^4$, $R^5$ et $R^6$ représentent chacun, indépendamment les uns des autres, un radical alkyle, cycloalkyle ou aralkyle ou un radical aryle à un ou deux noyaux, éventuellement porteur de 1 à 3 radicaux $-CH_3$, et les hydrocarbures cycliques comportant deux ou trois doubles liaisons et contenant de 6 à 12 atomes de carbone dans le cycle ou dans des systèmes bicycliques ou tricycliques, ou d'un mélange de ces formateurs de complexes, le rapport molaire de la composante a) à la composante b) étant compris entre 1 : 1 et 1 : 8.